# EUROPEAN PATENT APPLICATION

(11) **EP 2 974 667 A1**
(43) Date of publication of application: **20.01.2016**
(21) Application number: 15176394.3
(22) Date of filing: 13.07.2015
(51) Int. Cl.: A61B 10/00, A61B 5/107

(54) **A THICKNESS METER OF TISSUE OF THE HUMAN BODY**

(30) Priority: 14.07.2014 IT VI20140186; 01.04.2015 IT VI20150092
(71) Applicant: Marchesin, Stefano, 36100 Vicenza (IT)
(72) Inventor: Marchesin, Stefano, 36100 Vicenza (IT)
(74) Representative: Trentin, Michele

(57) **Abstract**

A thickness meter of tissues (T) of the human body susceptible to be coupled to an applicator for the projection of a beam of ionizing particles on tissues (T) of the body, comprising: a support body (2) having the base (3) susceptible to be arranged as support on the upper surface (S) of the tissues (T); a needle (5) associated with the support body (2) and movable so as to penetrate in the tissues (T); an indicating element (8) movable with the needle (5) with respect to the support body (2); measuring means (10) of the relative movement of the indicating element (8) with respect to the support body (2). The meter (1) has a shape that allows to insert it in the applicator for the projection of a beam of ionizing particles on tissues (T) of the human body so as to allow the measurement of the thickness of such tissues upon the positioning of the applicator.

## Description

### Field of Application

The present invention is applicable to the field of healthcare and, in particular, it relates to devices for the therapeutic treatment of patients.

More in detail, the present invention relates to devices for the cancerous therapeutic treatment.

### Background of the Invention

Among the most feared diseases of our time it is undeniable that cancer is believed to be prominent because of the mortality that some types of cancer have and the difficulty of therapy.

Tumour, or less commonly neoplasia, or cancer if it is malignant, it is a class of diseases characterized by uncontrolled reproduction of some body cells that stop answering to physiological mechanisms of cell control as a consequence of the damage of their genetic inheritance.

A cell, to become cancerous, must go crazy, that is there must be an error in the system that controls its reproduction.

The great speed of reproduction of cancerous cells is the basis of the need and urgency of treating it as soon as possible and as drastically as possible, that is by eliminating with the highest certainty degree as possible all "infected" cells because, as it has been said above, cancer may develop, and then born again, even from one single mutated cell.

The most known treatment for cancer is surgery. If possible, through it, it is tried to remove what is defined cancer mass, that is all the mutated cells, and what surrounds them. Such a method of treatment is not always usable or, when used, it is not sufficient to ensure the desired result. In fact, it is not possible to know if the cancer has affected even the surrounding, or adjacent, cells that seem healthy.

For such reasons, in combination to or as an alternative of the surgical intervention chemotherapy and radiotherapy are further used.

As far as the second treatment is concerned, it has been observed that the rapid reproduction of cancerous cells makes them particularly vulnerable to radiation with respect to the healthy tissues. This weakness is exploited to treat many kinds of solid cancer with radiotherapy, that is a bombardment with a beam of ionizing particles, in the attempt to kill the highest number of malignant or possible malignant cells.

However, such a treatment involves undesired effects. In particular, it significantly weakens the body and it subjects to its direct effects also the healthy organs of the patient, even if only partially. Therefore, it is important to minimize its application in order to not make the side effects too significant.

In this view, for some time now apparatuses are used for radiotherapy intraoperating treatment.

Radiotherapy intraoperating consists of an irradiation performed during the surgical removal of the cancerous mass. To perform it, a diffused and uniform beam of electrons is generally used as ionizing particles, having a predetermined kinetic energy and dependent on the thickness and features of the tissues to be treated. In particular, as a result of the surgical removal of the cancerous mass, the surrounding or adjacent tissues are submitted to radiation, particularly those where the cancerous mass laid thereon, to damage the cells and to prevent the repopulation of the cancerous cells.

In all cases wherein the disease does not have metastases, intraoperating radiotherapy may be applied as part of the total radiotherapy, or as a unique irradiation. Indeed, there is a large group of patients for whom the intraoperating irradiation totally and successfully replaces the necessary radiotherapy by drastically shortening the duration of the therapeutic phase.

The irradiation is accomplished by applying to the tissues to be irradiated a pipe of plastic material that guides and diffuses an electrons beam generated by an accelerator. The electrons penetrate in the tissues for few centimeters losing, with the penetration, their ionizing, and therefore therapeutic, power.

In FIG., it is schematically shown a typical situation of intraoperating irradiation in the case of removal of a mammary gland **M.**

The isodose lines **A** and **B** describe the way wherein the electronic beam deposits its energy and performs the therapeutic action. Usually, the area delimited by the isodose **A** of 90% is considered useful, which means that in this area the value of the deposited dose varies of 90 to 100%.

The volume concerned by the residual switching off, shown for reasons of clarity with the isodose **B** of 10%, does not have therapeutic effects.

Therefore, it is clear that an important factor for the success of the treatment relies on the measurement of the thickness of the tissues to be treated to adjust the physical and geometrical features of the beam of ionizing particles.

According to the prior art, the measurement is generally approximate in the sense that in certain cases a purely visual or empirical analysis is accomplished, thereby losing precision and undermining the result of the radiotherapy treatment.

Typically, moreover, the thickness of the tissues varies with the positioning of the applicator **P**, that is the guide pipe for the beam of ionizing particles, in the sense that such a pipe tends to press the tissues along its edge and to facilitate their bulging inwards the pipe thereof. Such a phenomenon makes the analysis of the effect of the beam on the tissues to be treated even more critical and, consequently, it makes the analysis of the most correct parameters for said beam more critical.

It is known the patent document US 2006/064039 A1 that describes a device for measuring the lumen of an organic duct. In this sense, it is structured to be inserted in such duct and not to measure the thicknesses by penetrating in the tissues.

Furthermore, it is known the patent document US 2,763,935 that actually is designed to measure the thickness of tissues of the human body. However, it is not designed to perform the measurement with the applicator **P** already in position, with the consequence that the measurements are not precise. Such an imprecision is not only due to the pressing of the tissues determined by the applicator **P**, but also to the lack of coaxiality between the meter and the applicator **P** that causes an additional measurement error.

### Summary of the Invention

Object of the present invention is to at least partially overcome the above mentioned drawbacks, by providing a thickness meter of tissues of the human body that allows to obtain such a measurement with precision.

Another object of the present invention is to provide a thickness meter of tissues of the human body that allows to perform such a measurement on multiple points so as the effect of the beam of ionizing particles is estimated with precision on the concerned area.

A further object is to provide a thickness meter of tissues of the human body that allows such measurements when the applicator is already positioned for the treatment.

Therefore, another object is to provide a thickness meter of tissues of the human body that is designed to be coupled to an applicator of beams of ionizing particles so as they are coaxial, that is by ensuring a substantially perfect measurement.

Such objects, and others which will appear more clearly hereinafter, are fulfilled by a thickness meter of tissues of the human body in accordance with the following claims that are an integral part of the present description.

In particular, the meter is susceptible to be coupled to an applicator for the projection of a beam of ionizing particles on tissues of the human body to determine the intensity of the beam of ionizing particles wherewith to radiate such tissues.

Firstly, it comprises a support body having the base susceptible to be arranged as a support on the upper surface of the tissues to be treated facing the source of the beam of ionizing particles.

Furthermore, the meter thereof comprises at least one needle associated with the support body and movable thereto so as to penetrate in the tissues until it protrudes from the surface opposite to the upper surface. At such a needle an indicating element is associated with, that is, therefore, movable with the needle with respect to the support body.

Then, there are measuring means, typically but not necessarily consisting of a graduated scale, of the relative movement of the indicating element with respect to the support body.

According to an aspect of the invention, the meter has a shape that allows its insertion inside the applicator.

Advantageously, therefore, the measurement of the thickness of the tissues is fulfilled by a special tool capable of giving a precise and non-empirical measurement.

Moreover, since it is obtained by measuring the difference in position between the tip of the needle and the base of the support body laying on the tissues, such a measurement has an excellent precision, ensuring an equally excellent result of the subsequent treatment.

Moreover, being the measurement performed once the applicator is positioned, it is precise since it implicitly considers the change of positioning of the tissues due to the positioning of the applicator thereof.

According to another aspect of the invention, the meter further comprises means of coaxial arrangement that allow to ensure the coaxiality of the meter to the applicator when it is inserted therein. This allows to ensure a substantially perfect precision of the performed measurement since the meter, when the measurement is performed, is positioned parallel to the beam of electromagnetic beams with which the tissues are irradiated.

As said, the objects are further fulfilled by an applicator for the projection of a beam of ionizing particles on tissues of the human body provided for at least one thickness meter of the tissues comprising:
- a support body having the base susceptible to be arranged as support on the upper surface of the tissues facing towards the source of the beam of ionizing particles;
- at least one needle associated with said support body and movable with respect to said support body so as to penetrate in the tissues until it protrudes from the surface opposite to the upper surface;
- at least one indicating element movable with said needle with respect to said support body;
- measuring means of said relative movement of said indicating element with respect to said support body.

Advantageously, therefore, the measurement of the thickness of tissues to be treated may be performed once the applicator is positioned. Since the latter tends to change the position of the tissues thereof and to press them, even though slightly, it is clear that in such a case the measurement is even more optimal and corresponding to reality, optimizing even more the effects of the treatment.

According to an aspect of the invention, means of the coaxial arrangement of the meter in the applicator are provided. Advantageously, this ensures the perfect measurement of the thickness of the tissues and, therefore, the optimization of the treatment.

### Brief description of the drawings

Further features and advantages of the invention will appear more clear upon reading the detailed description of some preferred, but not exclusive, embodiments of a thickness meter of tissues of the human body according to the invention, shown as non limitative example with the help of the annexed drawings wherein:
FIG. 1 represents a radiotherapy intraoperating treatment in schematic view;
FIG. 2 represents a meter according to the invention in perspective view;
FIG. 3 represents a detail of the meter of FIG. 2;
FIG. 4 represents an applicator according to the invention in perspective view.

### Detailed description of some preferred embodiments

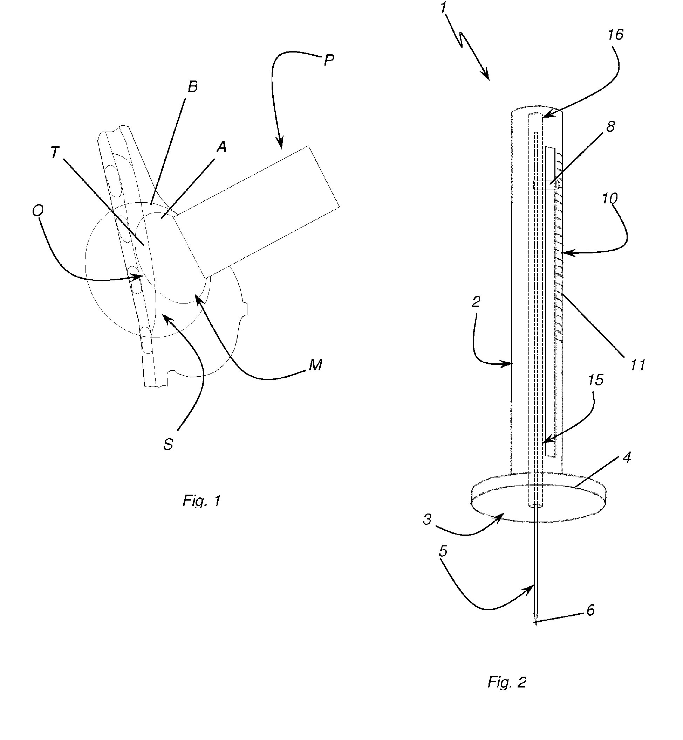

With reference to the above mentioned drawings, and particularly to FIGS. 1 and 2, it is described a thickness meter **1** of tissues **T** of the human body. Such a measurement, as said, is susceptible to be coupled to an applicator for the projection of a beam of ionizing particles on tissues **T** of the human body so as to determine the intensity of the beam of ionizing particles with which to irradiate the tissues **T** in the radiotherapy intraoperating treatment. As previously mentioned, such a measurement must be particularly precise so that the treatment has an optimal efficacy without excessively irradiating the patient.

According to an aspect of the invention, the meter **1** comprises a support body **2** whose base **3** is arranged, in the operating phase, as support on the upper surface **S** of the tissues **T** facing towards the source of the beam of ionizing particles.

In this way, the base **3** acts as reference for the subsequent measurement. It typically consists of a disk **4** so as to determine a valid support surface on the tissues **T** by correctly levelling them for a more effective measurement. Obviously, such an embodiment of the invention should not be limitative for different embodiments where the base of the support body is different from a disk or, nevertheless, from a plate-like equivalent element.

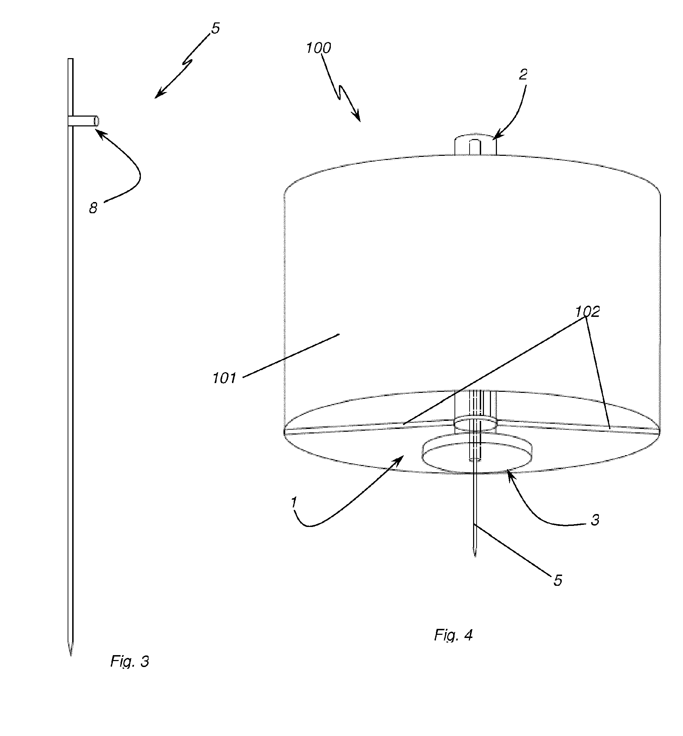

According to another aspect of the invention, the meter **1** further comprises a needle **5**, observable also in the detail of FIG. 3, associated with the support body **2** and movable thereto so as to penetrate in the tissues **T** until it protrudes from the opposite surface **O**. Therefore, it is evident that the differential between the tip **6** of the needle **5** and the base **3** of the support body **2** is, advantageously, the measurement of the thickness of the tissues **T**.

From this point of view, it is noted that, typically, in radiotherapy intraoperating treatments behind the tissues **T** to be treated suitable screens are arranged to avoid irradiating the underlying organs. Such screens perform a contrast for the tip **6** of the needle **5** ensuring that it does not excessively protrude from the tissues **T** invalidating the accuracy of the detection.

In order to easily detect the above mentioned differential, the meter **1** comprises an indicating element **8** movable with the needle **5** with respect to the support body **2**. The positioning of such an indicating element **8** is obviously designed in a way so as to make it easily observable during the measurement, that is, in the operating phase.

According to a further aspect of the invention, the meter **1** further comprises measuring means **10** of such relative movement of the indicating element **8** with respect to the support body **2**. In this way, not only the meter **1** allows to perform the measurement conveniently and in a safe way, but also, advantageously, it allows to perform a precise measurement.

According to the embodiment that is described, the measuring means **10** consist of a graduated scale **11**, but this detail should not be considered limitative for the invention.

As heretofore repeatedly underlined, the meter **1** has all the features designed to ensure a suitable and precise measurement of the thickness of the tissues **T** to be treated with radiotherapy. In this sense, according to another aspect of the invention the meter **1** further comprises a guide **15** for the needle **5** so as to ensure the coaxiality between the latter and the measuring means **10**. If the coaxiality is not ensured, it is obvious that the measurement is not correct.

According to the embodiment that is described, the guide **15** consists of a tubular portion **16** of the support body **2** wherein the needle **5** is free to slide. Such a tubular portion **16** flows into a passing-through opening made on the disk **4** at the base **3** of the support body **2** to allow the needle **5** to come out from the guide **15** and to penetrate in the underlying tissues **T**. However, even such an aspect of the invention should not be considered limitative for different embodiments according to which, for example, the needle is arranged externally to the support body and associated therewith by means of suitable rings. In this case, moreover, it is not required to pass through the disk on the base of the support body thereof.

An important aspect of the medical equipment is the safety of both operators and patients.

In this sense, the meter **1** of the invention comprises, although not shown in the drawings, a device for blocking the relative movement of the needle **5** with respect to the support body **2**. In the embodiment that is described it consists of a screw that, if tightened, presses against the shaft of the needle **5** stopping the movement, however, this is just an example of an embodiment of the invention.

Advantageously, such a blocking device allows to avoid unwanted movements of the needle **5**. In particular, both before and especially after the measurement, during the handling of the meter **1** by the operator the blocking device prevents the needle **5** to come out from the guide **15** in order to protect the operator from accidental injections.

Furthermore, still advantageously, after use the meter **1** must be subjected to sterilisation in order to be used with another patient. In such a case, the meter **1** is arranged inside suitable bags that are inserted in sterilising machines. Advantageously, the blocking device allows to prevent the needle **15** to move and to pierce the bags.

According to a possible embodiment, the needle is associated with the support body by means of a suitable rod where the needle thereof is movably associated therewith. Such a rod is susceptible to slide inside the guide and the length of the needle is such to avoid the rod coming in contact with the patient. In this case, to the object of sterilization both the needle and the disk at the base of the support body are removable from their seat in order to be sterilised and to avoid having to sterilize the entire meter.

Advantageously, therefore, the sterilising operation is simplified. Even the costs of implementation of the meter of the invention are diminished since both the support body and the rod may be made of not-biocompatible and less noble materials not having to be subjected to sterilisation. Therefore, they are also less expensive.

Moreover, such a embodiment advantageously allows to provide for the use of needles of different type, shape and sizes in accordance with the particular use.

As aforementioned, the radiotherapy intraoperating treatment is accomplished by means of the bombardment of tissues **T** with a beam of ionizing particles driven by an applicator **P** which is connected to a waveguide that is in its turn connected to the generator of such a beam.

In this sense, according to another aspect of the invention the meter **1** has a shape that allows to insert it in the applicator **P** so as to allow the measurement of the thickness of such tissues once the applicator **P** is positioned.

Advantageously, in the operating phase it is possible to proceed with the positioning of the applicator **P** and subsequently with the measurement of the thickness of the tissues **T** by inserting the meter **1** inside the applicator **P** thereof. It is clear that in this way the obtained measurement is precise because it refers to the actual operating situation where the applicator **P** inevitably changes the arrangement of the tissues **T**.

Moreover, since, typically, the tissues **T** tend to compress in correspondence with the perimeter of the applicator **P** and to protrude therein, it is evident that thickness of the tissues **T** varies from point to point. In such a case, still advantageously, the operator may proceed by performing the measurement at multiple points and by averaging the results, with obvious gain in the precision of the measurement and, therefore, with a better optimization of the treatment.

According to a further aspect of the invention, the coupling between the meter **1** and the applicator **P** is such as to maintain them reciprocally coaxial, so as to ensure the perfection of the measurement and the optimization of the radiotherapy treatment. In this sense, the meter comprises means of coaxial arrangement.

Typically they consist of a suitable shape of the support body **2** that, being arranged as support on the inner walls of the applicator **P**, ensures the coaxiality.

According to possible embodiments, the means of coaxial arrangement comprise shaped elements associated with the support body. Such shaped elements consist of, for example, a parallelepiped body with a triangular or polygonal base wherein the support body is arranged in a passing-through and coaxial way. Such elements are typically two associated with the ends of the support body so as that their contact with the inner walls ensures the coaxiality between the meter and the applicator.

Therefore, to what has been said it is evident that the invention is further an applicator **100**, observable in FIG. 4, for the projection of a beam of ionizing particles on tissues **T** of the human body. Such an applicator **100** is provided for at least one thickness meter **1** of tissues **T**.

As already described, the latter comprises a support body **2** having the base **3** susceptible to be arranged as a support on the upper surface **S** of the tissues **T** to be treated, a needle **5** associated with the support body **2** and respectively movable thereto, a indicating element **8** movable with the needle **5**, measuring means **10** of such a movement with respect to the support body **2**.

It is here omitted a complete discussion about the meter **1** since it would be repetitive of what has been said heretofore. However, what makes the applicator **100** particular is that it already incorporates one or more meters **1**.

As it is known, it comprises a substantially cylindrical body **101** susceptible to act as a guide for the beam of ionizing particles. As afore mentioned, means of coaxial coupling of the meter **1** in the applicator **100** are provided. This allows to ensure the coaxiality between the two of them and, therefore, the substantial perfection of the measurement.

Previously, some examples of embodiment of such means of coaxial arrangement have been described that, however, should not be considered limitative for the invention.

In fact, according to the particular embodiment shown in FIG. 4, the means of coaxial arrangement comprise arms **102** associated with the edge of the body **101** that hold in position the meter **1**.

Advantageously, therefore, the measurement is conveniently accomplished with the meter **1** held in position by the arms **102**. Moreover, the latter allow to maintain the coaxiality between the applicator **100** and the meter **1**, ensuring the precision of the measurement. Then, the **arms** 102 thereof are advantageously removable before the performance of the treatment so as not to influence it in any way.

Obviously, such features should not be considered limitative for different embodiments of the invention for which, for example, the number of meter is greater than one, the arms are not removable or are flexible to allow a repositioning of the meter. In this view, according to a particular embodiment, not shown in the annexed drawings, the side walls of the applicator have, in section, suitable seats wherein to house a plurality of meters that, in fact, perform measurements of minimum of the thickness of the tissues to be treated.

To what has been said, it is evident that a thickness meter of tissues of the human body and the applicator for the projection of a beam of ionizing particles on tissues of the human body of the invention both fulfil all the intended objects.

In particular, they allow to obtain precise measurements even on multiple points of the tissues so as the effect of the beam of ionizing particles may be estimated with precision on the concerned area. Such measurements may further be performed once the applicator is already positioned, improving the accuracy of the result.

The invention is susceptible of numerous modifications and variations, all falling within the appended claims. All the details may be replaced with other technically equivalent elements, and the materials may be different according to requirements, without departing from the scope of the invention defined by the appended claims.

## Claims

1. A thickness meter of tissues (**T**) of the human body susceptible to be coupled to an applicator for the projection of a beam of ionizing particles on tissues (**T**) of the human body to determine the intensity of the beam of ionizing particles wherewith to radiate such tissues (**T**), comprising:
- a support body (**2**) having the base (**3**) susceptible to be arranged on the upper surface (**S**) of the tissues (**T**) facing towards the source of the beam of ionizing particles;
- at least one needle (**5**) coupled to said support body (**2**) and movable with respect to said support body (**2**) so as to penetrate in the tissues (**T**) until it protrudes from the surface (**O**) opposite to the upper surface (**S**);
- at least one indicating element (**8**) movable with said needle (**5**) with respect to said support body (**2**);
- measuring means (**10**) of said relative movement of said indicating element (**8**) with respect to said support body (**2**),
**characterized in that** it has a shape that allows to insert said meter (**1**) into the applicator for the projection of a beam of ionizing particles on tissues (**T**) of the human body so as to allow the measurement of the thickness of such tissues upon the positioning of the applicator.

2. Meter according to claim 1, **characterized in** comprising means of coaxial arrangement that allow to ensure the coaxial arrangement of said meter in the applicator.

3. Meter according to claim 1 or 2, **characterized in that** said means of coaxial arrangement consist of the shape of said support body of said meter.

4. Meter according to any one of the preceding claims, **characterized in** comprising a guide (**15**) for said needle (**5**) so as to ensure the coaxiality with said measuring means (**10**).

5. Meter according to claim 4, **characterized in that** said guide (**15**) consists of a tubular portion (**16**) of said support body (**2**) where said needle (**5**) is free to slide therein.

6. Meter according to any one of the preceding claims, **characterized in** comprising a device for blocking the relative movement of said needle (**5**) with respect to said support body (**2**).

7. Meter according to any one of the preceding claims, **characterized in** comprising a disk (**4**) arranged in correspondence with said base (**3**) of said support body (**2**) so as to protect its support on the tissues (**T**).

8. Applicator for the projection of a beam of ionizing particles on tissues (**T**) of the human body provided for at least one thickness meter (**1**) of tissues comprising:
- a support body (**2**) having the base (**3**) susceptible to be arranged on the upper surface (**S**) of the tissues (**T**) facing towards the source of the beam of ionizing particles;
- at least one needle (**5**) associated with said support body (**2**) and movable with respect to said support body (**2**) so as to penetrate in the tissues (**T**) until it protrudes from the surface (**O**) opposite to the upper surface (**S**);
- at least one indicating element (**8**) movable with said needle (**5**) with respect to said support body (**2**);
- measuring means (**10**) of said relative movement of said indicating element (**8**) with respect to said support body (**2**),
**characterized in** comprising means for the coaxial arrangement of said meter (**1**) with said applicator (**P**).

9. Applicator according to claim 8, **characterized in that** said means for the coaxial arrangement consist of the shape of said support body (**2**).

10. Applicator according to claim 8, **characterized in that** the side walls of said cylindrical body have, in the thickness, one or more cavities where a respective said meter is arranged therein.
